# EUROPEAN PATENT SPECIFICATION

(11) **EP 3 042 948 B1**
(45) Date of publication and mention of the grant of the patent: **04.03.2020**
(21) Application number: 15184214.3
(22) Date of filing: 08.09.2015
(51) Int. Cl.: C12M 1/26, C12M 1/16

(54) **ROTARY TYPE SOLID CULTURE APPARATUS**
ROTIERENDE FESTSTOFF-KULTURVORRICHTUNG
APPAREIL DE CULTURE SOLIDE DE TYPE ROTATIF

(30) Priority: 07.01.2015 JP 2015001291
(43) Date of publication of application: 13.07.2016
(73) Proprietor: Fujiwara Techno-Art Co., Ltd., Okayama 701-1133 (JP)
(72) Inventor: WAKIMOTO, Katsumasa, Okayama-shi, Okayama 7011133 (JP); FUJIWARA, Keiko, Okayama-shi, Okayama 7011133 (JP)
(74) Representative: de Arpe Fernandez, Manuel

(56) References cited:
- EP-A2- 1 270 714
- CN-Y- 2 828 054

## Description

### TECHNICAL FIELD

Present invention relates to a discharger of a rotary type solid culture apparatus used for an enzymatic industry and a brewing industry such as a distilled spirit, a refined sake, miso, soy source, and others.

### BACKGROUND ART

In manufacturing processes of a brewing industry and an enzymatic industry such as koji making or others, a solid raw material is cultured with a rotary type solid culture apparatus. The solid raw material is fed into the rotary type solid culture apparatus, and the solid raw material after culturing is discharged out of the apparatus by a rotation of a discharging screw of the discharger. In case deposition thickness of the solid raw material is not even when feeding it, the thickness of the solid raw material is made even by rotating discharging screw in a normal direction or reverse direction. Normally, in a discharger of a rotary type solid culture apparatus, a rotating shaft supporting pipe going through a side wall moves up or moves down along the side wall to interchange a state in which the discharging screw is lowered on a solid raw material side and a state which the discharging screw is lifted above the solid raw material.

In order to realize this up-and-down movement, the number of parts becomes large and a structure becomes complicated since an up-and-down motor, a speed reducer, a counter shaft, a threaded rod, a slide rod positioning a supporting arm and others are required. Furthermore, it is difficult to completely seal the outer air since the rotating shaft supporting pipe which goes through the side wall and moves up or down needs a sealing member.

Since a tank rotating type koji making apparatus disclosed in Patent Literature 1 below is provided with a motor of rotating a discharging screw inside the apparatus, a portion going through the side wall and moving up or down is not provided. Therefore, a sealing performance would be enhanced. However, it does not simplify a structure since it requires a wire drum and others for moving the discharger up or down.

Contrast to this, a rotary type solid culture apparatus disclosed in Patent Literature 2 below is provided with a swing type agitator, which interchanges a state in which the discharging screw is lowered on a solid raw material side and a state in which the discharging screw is lifted above the solid raw material by swinging, not by up or down motion of a rotation shaft. CN 2 828 054 Y discloses a koji making device having a discharging screw type conveyor which can be moved up and down.

### Prior Art Documents

### Patent Literature

Patent Litrature1: JP 55-51546 B
Patent Literature 2: JP 06-153906 A

### SUMMARY OF THE INVENTION

### Problem to be Solved by the Invention

However, one disclosed in Patent Literature 2 above is the agitator which is different from a discharger in terms of a use and used state. Namely, the discharger is provided with a back board and a scraper, which the agitator does not have, and conducts a task evening a surface of a raw material, which is not conducted with the agitator. If the swing system disclosed in Patent Literature 2 is merely applied to the discharger, since the discharger is provided with the back board, problems arise such as a raw material remaining at the back board, the back board pressing and crushing the raw material, the back board interfering with evening of a deposition thickness, the scraper interfering with a center cylinder.

Object of the present invention is to solve previous problems described above, and to provide a rotary type solid culture apparatus in which a sealing performance of a discharger is enhanced and a simplicity of configuration are realized and in which a swinging structure is adopted without impairing functions of a discharger.

### Means to Solve the Problems

Provided is a rotary type solid culture apparatus to achieve the object. The rotary type solid culture apparatus provided with a discharger discharging a raw material outside the apparatus, the discharger including; a discharging screw; a swing shaft making the discharging screw swing; and an arm supporting the discharging screw, and rotating integrally with the swing shaft. The discharging screw swings and displaces between a state in which the discharging screw is lowered on a raw material side and a state in which the discharging screw is lifted above the raw material. The discharger further including; a back board covering one side of the discharging screw along an axial direction of the discharging screw; and a linkage connecting between the swing shaft and the back board. A positional relationship in which the back board is vertical with respect to the raw material is maintained by the linkage when the discharging screw swings and displaces.

According to this configuration, since the discharging screw is able to swing and displace between a state in which the discharging screw is lowered on a raw material side and a state in which the discharging screw is lifted above the raw material without vertically moving the swing shaft up or down, a mechanism for moving up or down is not required to simplify a configuration. Further, since the swing shaft does not move up or down, a sealing structure blocking the outer air is simplified and a sealing performance is enhanced even though it adopts a configuration that the swing shaft goes through the side wall of the apparatus. Still further, since it is provided with the linkage, the raw material is prevented from being remained at the back board when the discharging screw is lifted from a state in which the discharging screw is lowered on the raw material side. It prevents the back board from contacting to the raw material before the discharging screw does. This prevents the back board from interfering with the evening of the deposition thickness of the raw material conducted by the discharging screw, and prevents the back board from pressing and crashing the raw material. Further, it prevents the scraper guiding the raw material around the center cylinder to the discharging screw from interfering with the center cylinder.

It is preferable that the rotary type solid culture apparatus further includes a multi shaft structure configured by a main shaft accommodating the swing shaft and a rotary shaft to rotate the discharging screw, by the multi shaft structure, the discharger is driven and swung, and the discharging screw is driven and rotated, respectively. According to this configuration, since respective shafts transmitting two kinds of rotative forces are integrated to the multi shaft structure, a configuration of a transmission mechanism of the rotative forces is simplified and downsized.

According to the present invention, since the discharging screw is able to swing and displace between a state in which the discharging screw is lowered on a raw material side and a state in which the discharging screw is lifted above the raw material without vertically moving the swing shaft up or down, a mechanism for lifting or lowering is not required to simplify a configuration. Further, since the swing shaft does not lift or descend, a sealing structure blocking the outer air is simplified and a sealing performance is enhanced even though it adopts a configuration that the swing shaft goes through the side wall of the apparatus. Still further, since it is provided with the linkage, the raw material is prevented from being remained at the back board when the discharging screw is lifted from a state in which the discharging screw is lowered on the raw material side. Since it prevents the back board from contacting to the raw material before the discharging screw does, this prevents the back board from interfering with the evening of the deposition thickness of the raw material conducted by the discharging screw, and prevents the back board from pressing and crashing the raw material. Further, it prevents the scraper guiding the raw material around the center cylinder to the discharging screw from interfering with the center cylinder.

### BRIEF DESCRIPTION OF THE DRAWINGS

Fig. 1 is a vertical cross-sectional view illustrating a rotary type solid culture apparatus which is an embodiment of the present invention;
Fig. 2 is a plain view illustrating a main part of the rotary type solid culture apparatus illustrated in Fig. 1;
Fig. 3 is an enlarged plain view illustrating a vicinity of a discharger which is an embodiment of the present invention;
Fig. 4 is an enlarged vertical cross-sectional view illustrating a vicinity of a discharger which is an embodiment of the present invention;
Fig. 5 is a side view of a main part illustrating a state in which the discharger is discharging a raw material according to an embodiment of the present invention;
Fig. 6 is a side view of a main part illustrating a state of a discharger during a raw material is being cultured according to an embodiment of the present invention;
Fig. 7 is a side view of a main part illustrating a motion of a discharger which is not provided with a linkage;
Fig. 8 is a side view of a main part illustrating a motion of another discharger which is not provided with a linkage;
Fig. 9 is an enlarged plain view illustrating a vicinity of the discharger illustrated in Fig. 8.

### EMBODIMENTS FOR CARRYING OUT THE INVETION

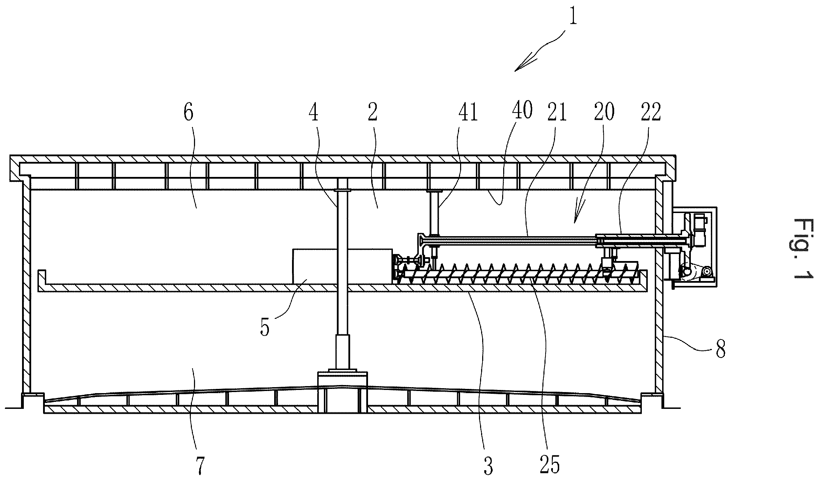

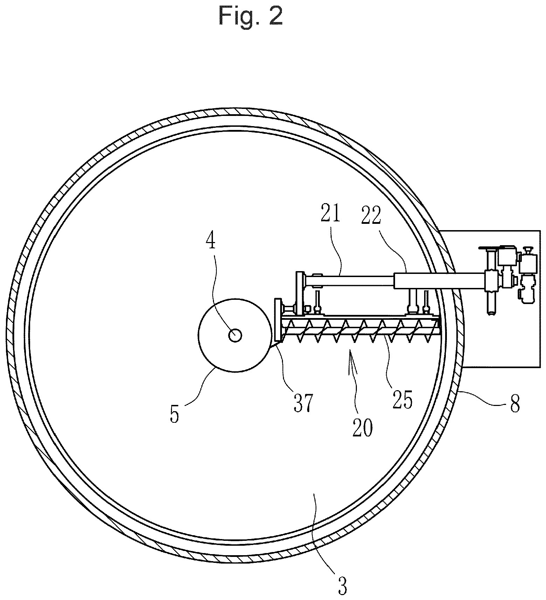

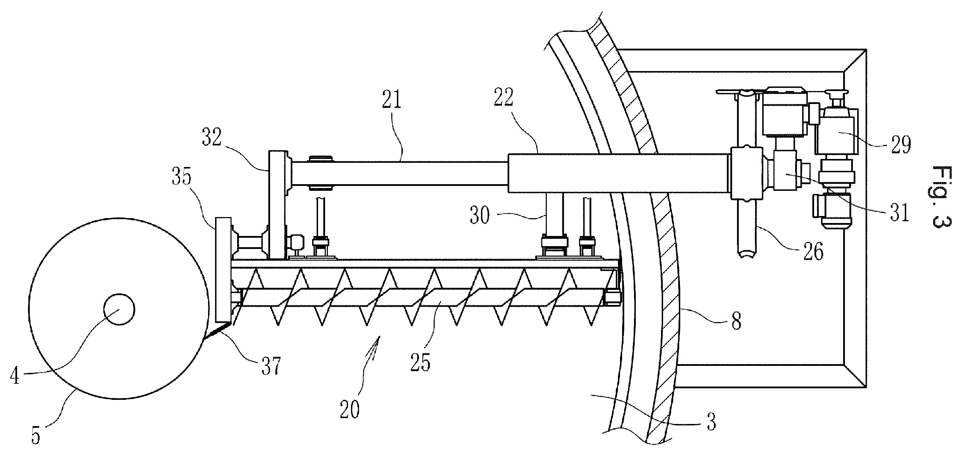

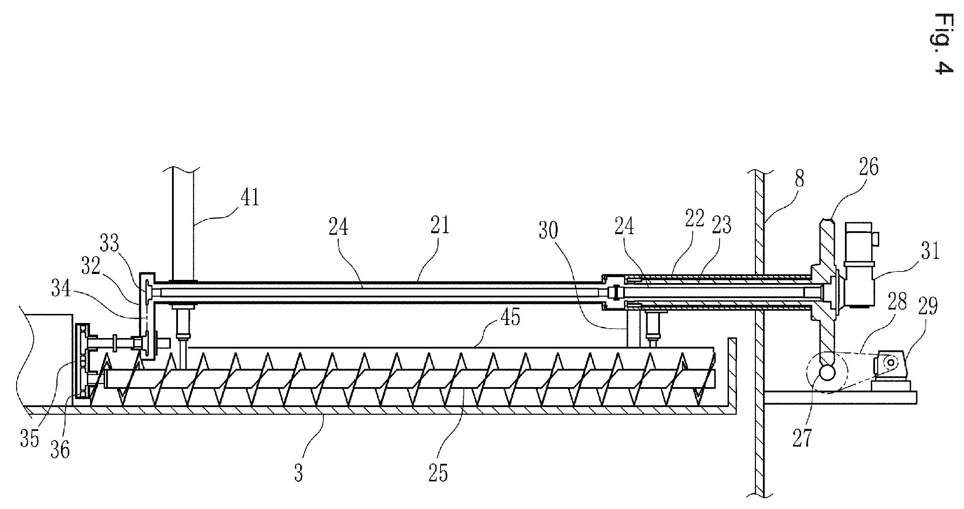

Hereinafter, a rotary type solid culture apparatus which is one embodiment of the present invention is explained with reference to the Figs. Fig. 1 is a vertical cross-sectional view illustrating a rotary type solid culture apparatus 1 which is an embodiment of the present invention. Fig. 2 is a plain view illustrating a main part of the rotary type solid culture apparatus 1 illustrated in Fig. 1. Fig. 3 is an enlarged plain view illustrating a vicinity of a discharger 20. Fig. 4 is an enlarged vertical cross-sectional view illustrating a vicinity of a discharger 20.

In Fig. 1, a disk shaped incubation bed 3 is provided inside an incubation cell 2 which is thermally insulated. The disk shaped incubation bed 3 rotates about a center support pillar 4 as a center. An inside of the incubation cell 2 is divided into an upper cell 6 and a lower cell 7. A conditioned air flow, which is flowed into the lower cell 7 from an air conditioner (not illustrated), flows into an inside of the upper cell 6 through a raw material (solid state culture material) on the disk shaped incubation bed 3. According to this, the raw material on the disk shaped incubation bed 3 is cultured while the conditioned air passes therethrough.

The raw material on the disk shaped incubation bed 3 after culturing is discharged to the outside of the apparatus with a discharger 20. As explained later in detail, a discharging screw 25 of the discharger 20 swings and displaces between a state in which the discharging screw is lowered on a raw material side and a state in which the discharging screw is lifted above the raw material. Fig. 1 illustrates the state in which the discharging screw 25 is lowered on a raw material (not illustrated) side. The discharging screw 25 rotates in this state and discharges the raw material on the disk shaped incubation bed 3 to the outside of the apparatus. During this time, the disk shaped incubation bed 3 rotates about the center support pillar 4 as the center to serially supply the raw material to the discharging screw 25. As illustrated in Fig. 2 and Fig. 3, since tip of the scraper 37 made of plastic mounted on the discharger 20 contacts to a center cylinder 5, the scraper 37 guides the raw material around the center cylinder 5 to the discharging screw 25 and prevents the raw material from adhering to a side face of the center cylinder 5 at the same time.

As illustrated in Figs. 1 and 4, a main shaft 21 of the discharger 20 is supported with a main shaft support arm 41 hanging from a ceiling 40 on a center side of the disk shaped incubation bed 3, and is supported with a main shaft support pipe 22 going through the side wall 22 on an outer side of the disk shaped incubation bed 3. As illustrated in Fig. 4, a pipe shaped swing shaft 23 and a rotation transmission shaft 24 are provided inside the main shaft support pipe 22. Namely, since two kinds of shafts of the swing shaft 23 and the rotation transmission shaft 24 which transmit respective rotative forces are integrated to the multi shaft structure, a configuration of a transmission mechanism of the rotative forces is simplified and downsized.

In Fig. 4, a worm wheel 26 is provided on one side of the swing shaft 23. The worm wheel 26 is connected to a swing motor 29 via a worm 27 and chain 28. The other end of the swing shaft 23 retains the discharging screw 25 via the arm 30. The discharging screw 25 is supported by the main shaft 21 via a chain case 32 and a gear case 35 on the center side of the disk shaped incubation bed 3. According to this configuration, if the swing motor 29 operates, the worm wheel 26 rotates via the chain 28 and the worm 27. The swing shaft 23 rotates integrally with the worm wheel 26. This rotation makes the arm 30 rotate. The discharging screw 25 swings integrally with the arm 30 about the swing shaft 23 as a center.

As illustrated in Fig. 4, a discharging screw rotation motor 31 is connected to a rotation transmission shaft 24. The rotation transmission shaft 24 is connected to a sprocket 33 accommodated in a chain case 32 on a center side of the disk shaped incubation bed 3. The sprocket 33 is connected to a gear 36 driven by a chain 34 and accommodated in the chain case 32. The gear 36 is connected with the discharging screw 25. According to this configuration, if the discharging screw rotation motor 31 operates, the rotation transmission shaft 24 rotates, this makes the sprocket 33 and the gear 36 integrally rotate and furthermore the discharging screw 25 rotates. According to this, the raw material on the disk shaped incubation bed 3 is discharged to the outside of the apparatus while agitated.

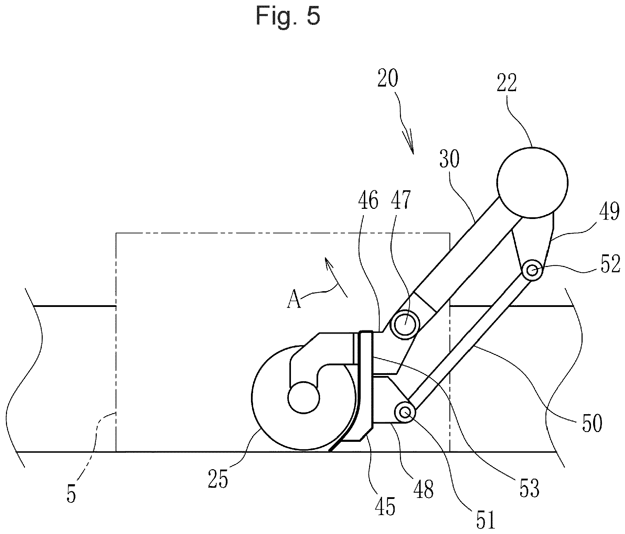

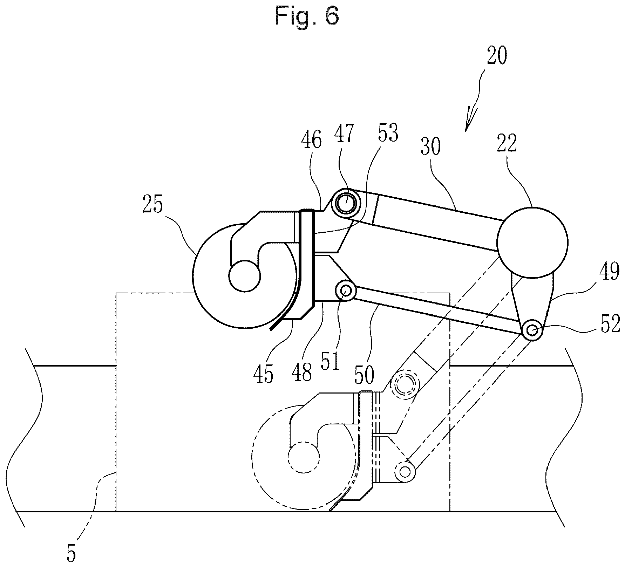

The discharging screw 25 which swings and displaces is explained with reference to Figs. 5 and 6. Fig. 5 is a side view of a main part illustrating a state in which the discharger 20 is discharging a raw material (not illustrated). The discharging screw 25 is buried in the raw material and rotates to discharge the raw material in this state. The portions illustrated with solid lines in Fig. 6 illustrates a state in which the raw material (not illustrated) is being cultured. The discharging screw 25 is lifted above the raw material in this state. For a convenience, a state in which the discharging screw 25 is buried in the raw material (a state of Fig. 5) is illustrated with two-dot chain lines.

Hereinafter, a lifting mechanism of the discharging screw 25 and the back board 45 is explained with reference to Fig. 5. One side of the discharging screw is covered with the back board 45. As illustrated in Fig. 4, the back board 45 covers one side of the discharging screw 25 along an axis direction of the discharging screw 25. As illustrated in Fig. 5, a support member 46 supporting an arm 30 is fixed to the back board 45. One end of the arm 30 is fixed to the support member 46 via a shaft 47. As illustrated in Fig. 4, the other end of the arm 30 is fixed to the swing shaft 23. The arm 30 rotates in sync with a rotation of the swing shaft 23.

As illustrated in Fig. 5, a support member 48 is fixed to the back board 45, and a support member 49 is fixed to a main shaft support pipe 22. One end of a connecting member 50 is fixed to the support member 48 via a shaft 51. The other end of the connecting member 50 is fixed to the support member 49 via a shaft 52.

In a state of Fig. 5 in which the discharging screw 25 is lowed on the raw material side, as explained above, if the swing motor 29 operates, the worm wheel 26 rotates via the chain 28 and the worm 27 and the swing shaft 23 rotates integrally with the worm wheel 26. This rotation makes the arm 30 rotate. As illustrated in Fig. 5, the arm 30 is connected to the support member 46 fixed to the back board 45 via the shaft 47. Therefore, if the arm 30 rotates upwardly (direction indicated with arrow A), the discharging screw 25 and the back board 45 lift to a position indicated with the solid lines in Fig. 6 integrally therewith.

An upper stopping position and a lower stopping position where the lowering or lifting discharging screw 25 and the back board 45 stop can be set by providing a limit plate on the worm wheel 26 (Fig. 4) and providing a limit switch on this striker plate. A photoelectric switch is usable in place of the limit switch.

If the swing motor 29 illustrated in Fig. 4 operates in a reverse direction in a state in which the discharging screw 25 and the back board 45 are lifted at the position indicated with the solid lines in Fig. 6, the discharging screw 25 and the back board 45 lowers to the position indicated with two-dot chain lines (position indicated in Fig. 5).

As explained above, according to the present embodiment, the discharging screw 25 swings and displaces between a state in which the discharging screw 25 is lowered on the raw material side and a state in which the discharging screw 25 is lifted above the raw material side without vertically moving up or down the swing shaft 23. Therefore, a mechanism for moving up or down is not required to simplify a configuration. Further, since the swing shaft 23 does not move up or down, a sealing structure blocking the outer air is simplified and a sealing performance is enhanced even though it adopts a configuration that the swing shaft 23 goes through the side wall 8 of the apparatus 1 as illustrated in Fig. 4.

Herein, a connecting structure between the swing shaft 23 (Fig. 4) and the back board 45 is a planar linkage in the discharger 20 explained with reference to the above explained Fig. 5. An electing portion 53 of the back board 45 electing vertically corresponds to a side of a parallelogram configuring the planar linkage. This makes the electing portion 53 parallel displace to keep a positional relationship in which the back board 15 is vertical with respect to the raw material if the arm 30 and the connecting member rotate 50 and move. According to this configuration, since the back board 45 does not incline when the discharging screw 25 lifts, it prevents the raw material from being remained at the back board 45.

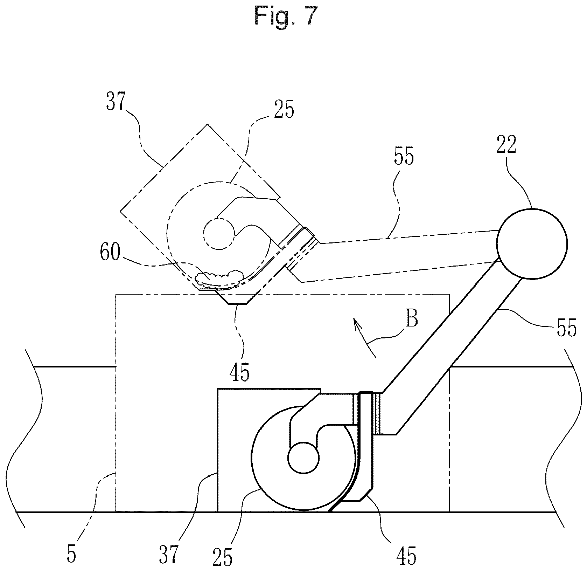

It is compared with a configuration of Fig. 7 in terms of a prevention of remaining raw material. Fig. 7 is a side view of a main part illustrating a motion of a discharger which is not provided with a linkage as a connecting structure between a swing shaft 23 (Fig. 4) and the back board 45. An arm 55 and a back board 45 are directly connected without intervening a shaft. Solid lines in Fig. 7 illustrates a state in which a discharging screw 25 is lowed on the raw material side. Two-dot chain lines illustrates a state in which the discharging screw 25 finishes lifting. If the arm 55 rotates upwardly from a state in which the discharging screw 25 is at the position of the solid lines (direction indicated with arrow B), the back board 45 and the discharging screw 25 lift integrally with the arm until they reach the position of the two-dot chain lines. While lifting, a vertical positional relationship with respect to the raw material is not maintained. Inclining angle of the back board 45 gradually increases as it lifts. In this case, the raw material 60 remains at the back board 45 as illustrated in Fig. 7.

According to the configuration of Fig. 7, if the back board 45 and the discharging screw 25 are made lowered from the positon of the two-dot chain lines, the back board 45 contacts the raw material before the discharging screw 25 does. Contrast to this, according to the configuration of Fig. 5 and Fig. 6, the back board 45 maintains a vertical positional relationship with respect to the raw material even though the back board 45 and the discharging screw 25 are made lowered from a position of the solid lines to the positon of the two-dot chain lines. Therefore, the back board 45 is prevented from contacting the raw material before the discharging screw 25 does. This prevents the back board 45 from interfering with the evening of the deposition thickness of the raw material conducted by the discharging screw 25, and prevents the back board 45 from pushing and crashing the raw material.

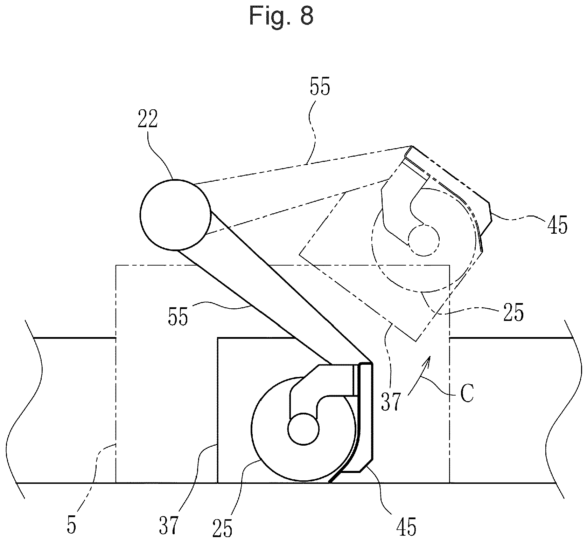

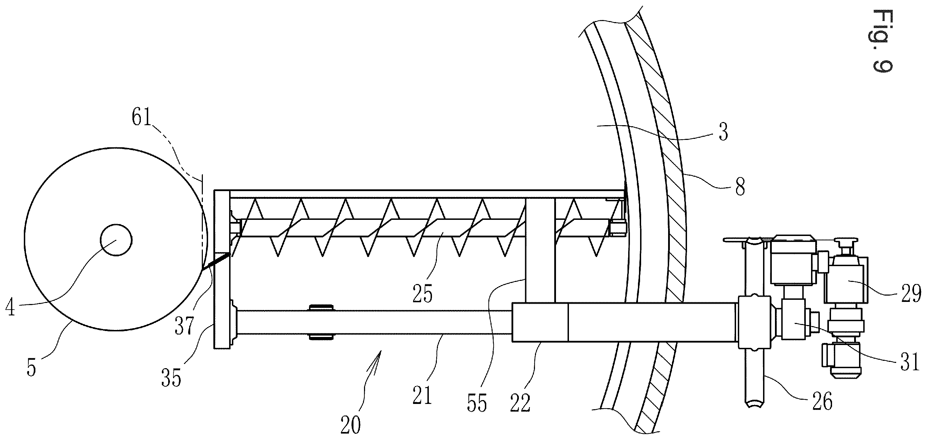

A measure for preventing the raw material from being remained is taken in a discharger illustrated in Fig. 8. Fig. 9 is an enlarged plain view illustrating a vicinity of the discharger illustrated in Fig. 8. According to a configuration of Fig. 8, when a discharging screw 25 lifts (in a direction indicated with arrow C), the back board 45 lifts in advance of the discharging screw 25. Since a plane of the back board on a discharging screw side faces lower side, the raw material is prevented from remaining.

However, according to the configuration of Fig. 8, a problem of which a scraper 37 interferes with a side face of a center cylinder 5 arises. Concretely, as illustrated in Fig. 8, the discharging screw 25 is made swing between the solid lines and two-dot chain lines, an orbit of the scraper 37 is on a line 61 as illustrated in Fig. 9 and the scraper 37 and the center cylinder 5 interfere each other. In contrast to this, according to the configuration of Fig. 6, the back board maintains a vertical positional relationship with respect to the raw material even though the back board 45 and the discharging screw 25 swing between the solid lines and the two-dot chain lines. An electing state of the scraper 37 is maintained as well. Since the scraper 37 lifts and descends along a side face of the center cylinder 5, the scraper 37 is prevented from interfering with the center cylinder 5.

As compared the configuration of Fig. 6 provided with the linkage and the configurations of Fig. 7 and Fig. 8 which are not provided with the linkage, all of the problems of the remaining raw material, the back board 45 contacting the raw material before the discharging screw 25, the interfering scraper 37 are not solved by a configuration which merely swings the discharging screw 25. However, the present embodiment solves all of the problems by providing the linkage.

### Reference Numerals

- 1: rotary type solid culture apparatus
- 3: disk shaped incubation bed
- 4: center support pillar
- 5: center cylinder
- 8: side wall
- 20: discharger
- 21: main shaft
- 22: main shaft support pipe
- 23: swing shaft
- 24: rotation transmission shaft
- 25: discharging screw
- 30: arm
- 37: scraper
- 45: back board
- 50: connecting member

## Claims

1. A rotary type solid culture apparatus provided with a discharger (20) discharging a raw material outside the apparatus (1), the discharger comprising;
a discharging screw (25);
a swing shaft (23) making the discharging screw swing; and
an arm (30) supporting the discharging screw, and rotating integrally with the swing shaft: wherein
the discharging screw (25) swings and displaces between a state in which the discharging screw is lowered on a raw material side and a state in which the discharging screw is lifted above the raw material by a swing of the arm (30):
the discharger further (25) comprising; a back board (45) covering one side of the discharging screw along an axial direction of the discharging screw; and a linkage connecting between the swing shaft (23) and the back board (45); wherein
a positional relationship in which the back board (45) is vertical with respect to the raw material is maintained by the linkage when the discharging screw (25) swings and displaces.

2. The rotary type solid culture apparatus according to Claim 1, further comprising a multi shaft structure configured by a main shaft (21) accommodating the swing shaft (23) and a rotary shaft to rotate the discharging screw (25);
wherein by the multi shaft structure, the discharger is driven and swung, and the discharging screw (25) is driven and rotated, respectively.

## Patentansprüche

1. Ein kompaktes Landwirtschaftsgerät vom Ratationstyp ausgerüstet mit einem Auslader (20), der ein Rohmaterial ausserhalb des Gerätes (1) entlädt, wobei der Auslader (20) Folgendes einschliesst:
Eine Entladeschnecke (25);
Eine Spindelachse (23), die die Entladeschnecke dreht; und
Einen Arm (30), der die Entladeschnecke trägt und zusammen mit der Spindelachse dreht, wobei
die Entladeschnecke (25) dreht und sich zwischen einer Lage, bei der die Entladeschnecke sich in Richtung des Rohmaterials senkt, und einer Lage, in der sie sich über dem Rohmaterial durch eine Drehung des Arms (30) hebt;
der Auslader (20) umfasst weiter ein Stützelement (45), das eine Seite der Entladeschnecke in axialer Richtung der Entladeschnecke verdeckt, und eine Verbindung zwischen der Spindelachse (23) und dem Stützelement (45); wobei
eine gegenseitige Position, bei der das Stützelement (45) mit Bezug auf das Rohmaterial vertikal ist, wird durch die Verbindung gehalten, wenn die Entladeschnecke (25) sich dreht und die Stellung wechselt.

2. Das kompakte Landwirtschaftsgerät vom Ratationstyp gemäss Anspruch 1, das weiterhin eine Mehrachsenstruktur umfasst gebildet aus einer Hauptachse (21), die die Spindelachse (24) aufnimmt, und einer Drehachse für die Drehung der Entladeschnecke (25),
wobei der Auslader durch die Mehrachsenstruktur angetrieben und gedreht wird, und die Entladeschnecke (25) entsprechend angetrieben und gedreht wird.

## Revendications

1. Un appareil de culture solide de type rotatif avec un déchargeur (20) pour décharger de la matière première à l'extérieur de l'appareil (1), ledit déchargeur comprenant:
une vis à déchargement (25);
un arbre oscillant (23) qui fait osciller la vis de déchargement; et
un bras (30) pour supporter la vis à déchargement et effectuer la rotation intégrale avec l'arbre oscillant; où
la vis à déchargement (25) oscille et se déplace d'une position où la vis à déchargement est baissée sur le côté de la matière première jusqu'à une position où la vis à déchargement est élevée au-dessus de la matière première par une oscillation du bras (30) :
le déchargeur (20) comprend en outre une planche arrière (45) qui couvre un côté de la vis à déchargement le long d'une direction axiale de ladite vis à déchargement; et une articulation qui connecte l'arbre oscillant (23) et la planche arrière (45); où
une relation de position dans laquelle ladite planche arrière (45) est verticale par rapport à la matière première est maintenue par l'articulation lorsque la vise de décharge (25) oscille et se déplace.

2. L'appareil de culture solide de type rotatif conformément à la revendication 1, qui comprend en outre une structure multi-arbres configurée par un arbre principal (21) qui intègre l'arbre oscillant (23) et un arbre rotatif pour faire tourner la vis à déchargement (25);
où, par la structure multi-arbre, le déchargeur est actionné et oscillé, et la vis à déchargement (25) est actionnée et tournée, respectivement.
